# EUROPEAN PATENT APPLICATION

(11) **EP 4 285 832 A1**
(43) Date of publication of application: **06.12.2023**
(21) Application number: 22197376.1
(22) Date of filing: 23.09.2022
(51) Int. Cl.: A61B 6/12, A61B 6/00, A61B 8/12

(54) **GUIDING AN INTERVENTIONAL IMAGING DEVICE**

(30) Priority: 01.06.2022 US 202263347692 P
(71) Applicant: Koninklijke Philips N.V., 5656 AG Eindhoven (NL)
(72) Inventor: SINHA, Ayushi, Eindhoven (NL); LEE, Brian C., Eindhoven (NL); VARBLE, Nicole, 5656AG Eindhoven (NL)
(74) Representative: Philips Intellectual Property & Standards

(57) **Abstract**

The present invention relates to a device for guiding an interventional imaging device. In order to minimize the use of modalities like X-ray or CT, a device (10) for guiding an interventional imaging device is provided. The device comprises a data input (12), a data processor (14) and an output interface (16). The data input is configured to provide first image data as first data from a first imaging device. The first image data comprises a representation of the interventional imaging device inserted within a vessel structure of a subject. The first image data comprises image data relating to a first point in time. The data input is also configured to provide second data relating to a movement of the interventional imaging device. The second data relates to the first point in time and to at least a second point in time. The data processor is configured to estimate a pose of the interventional imaging device in the first image data. The data processor is also configured to track a relative motion of the interventional imaging device based on the second data. The data processor is further configured to compute an updated pose estimate of the interventional imaging device based on the estimated pose and the tracked relative motion. The data processor is furthermore configured to generate an updated indicator of the interventional imaging device based on the computed updated pose estimate. The data processor is also configured to augment the first image data with the updated indicator. The output interface is configured to provide the augmented first image data. In an example, the first imaging device is an X-ray imaging device and the second imaging device is a bronchoscopy imaging device.

## Description

### FIELD OF THE INVENTION

The present invention relates to guiding an interventional imaging device. The present invention in particular relates to a device for guiding an interventional imaging device, to a system for guiding an interventional imaging device and to a method for guiding an interventional imaging device.

### BACKGROUND OF THE INVENTION

In minimally invasive procedures, imaging devices are used that can be inserted into the subject body to help navigate to the region of interest (ROI) and/or to image the ROI. Examples include devices with visible light (RGB) cameras (endoscopes, bronchoscopes, etc.) as well as other imaging modalities such as endobronchial ultrasound (EBUS), intravascular ultrasound (IVUS), optical coherence tomography (OCT) or the like. They can be used in combination with other interventional imaging modalities like X-ray, tomosynthesis, cone-beam computed tomography (CBCT) and the like that can provide further support in navigation. However, it has been shown that for providing information about the inserted interventional devices using further data sources like an external X-ray imaging system increases exposure to ionizing radiation.

### SUMMARY OF THE INVENTION

There may thus be a need to minimize the use of these modalities.

The object of the present invention is solved by the subject-matter of the independent claims; further embodiments are incorporated in the dependent claims. It should be noted that the following described aspects of the invention apply also for the device for guiding an interventional imaging device, for the system for guiding an interventional imaging device and for the method for guiding an interventional imaging device.

According to the present invention, a device for guiding an interventional imaging device is provided. The device comprises a data input, a data processor and an output interface. The data input is configured to provide first image data as first data from a first imaging device. The first image data comprises a representation of the interventional imaging device inserted within a vessel structure of a subject, and the first image data comprises image data relating to a first point in time. The data input is also configured to provide second data relating to a movement of the interventional imaging device. The second data relates to the first point in time and to at least a second point in time. The data processor is configured to estimate a pose of the interventional imaging device in the first image data. The data processor is also configured to track a relative motion of the interventional imaging device based on the second data. The data processor is further configured to compute an updated pose estimate of the interventional imaging device based on the estimated pose and the tracked relative motion. The data processor is furthermore configured to generate an updated indicator of the interventional imaging device based on the computed updated pose estimate. And the data processor is configured to augment the first image data with the updated indicator. The output interface is configured to provide the augmented first image data.

In an example use case, the first image data may be X-ray images or fluoroscopy sequences acquired from an X-ray imaging system and the second image data maybe bronchoscopy images acquired from a bronchoscope as it is navigated within the lung airway anatomy. The imaging systems are used in combination during endobronchial procedures, where interventional pulmonologists navigate bronchoscopes under fluoroscopy guidance to lung lesions in order to biopsy or treat the lesions. As an effect of the present invention, beneficial for the subject, interventional pulmonologist and other staff, the use of the bronchoscope in navigation to the lesion is maximized and the use of fluoroscopy is minimized.

As an advantage, video-based bronchoscope tracking, does not require any additional hardware or change in workflow. Contrary, technologies like shape sensing and electromagnetic navigation, which can also be used to track bronchoscopes, require additional hardware, for example an electromagnetic field generator, electromagnetic tracked tools or shape sensed catheters and the like, which can be expensive and can add additional workflow steps for incorporation in the procedure. As another advantage, it is avoided that interventional pulmonologists or other users need to pinpoint their location in the patient anatomy from only looking at bronchoscopy images, which may disorienting for the users. These advantages are also applicable to other endoscopy, colonoscopy and other video-based procedures.

According to an example, the second data is image data from a second imaging device provided by the interventional imaging device. In other words, the interventional imaging device comprises the second imaging device. The image data from the second imaging device can also be referred to as second image data. The second image data comprises a representation of the interior within the vessel structure. The data processor is configured to track the relative motion of the interventional imaging device within the vessel structure based on the second image data.

According to an example, for the estimation of the pose of the interventional imaging device in the first image data, the data processor is configured to use images of the second image data, i.e. one or more of the images of the plurality of images of the second image data, used to generate pose estimates relating to at least the first point in time for adapting the estimated pose of the interventional imaging device in the first image data.

The second images, e.g. bronchoscope images, are used to estimate the pose, based on the image content by e.g. image processing or image analysis procedures.
According to an example, the second image data comprises a stream of second images. The data processor is configured to provide the tracking of the relative motion of the interventional imaging device for consecutive images of the stream of second images.

As an example, during endobronchial biopsy procedures, both bronchoscopy and fluoroscopy images are used to navigate physicians to pulmonary lesions.

As an example, machine learning systems are provided that can track cameras through space with reasonable accuracy based only on the video captured by the cameras, such as videos captured by a bronchoscope.

According to an example, the data processor is further configured to compute a trajectory of the interventional imaging device based on the updated pose estimate. In a first option, the data processor is configured i) to generate, based on the computed trajectory and the updated pose of the interventional imaging device, a projection of the trajectory of the interventional imaging device. The data processor is also configured to augment the first image data based on the projection of the trajectory of the interventional imaging device to provide an updated virtual first image data. In a second option, the data processor is configured ii) to project the computed trajectory onto the first image data.

According to an example, the data processor is further configured to use a trained generative neural network to generate, based on the first image data at the first point in time and an updated pose of the interventional imaging device at a second point in time, a realistic synthetic image rendering the updated pose of interventional imaging device.

According to an example, the data processor is further configured to provide a confidence estimate related to the relative motion estimate from the second image data. The output interface is configured to provide a confidence indicator to the user.

According to the present invention, also a system for guiding an interventional imaging device is provided. The system comprises a first data arrangement comprising a first imaging device. The system also comprises a second data arrangement and a device for guiding an interventional imaging device according to any of the preceding examples. The first imaging device is configured to generate the first image data as the first data. The second data arrangement is configured to generate the second data.

According to an example, an interventional imaging device is provided. In an option, the second data arrangement is provided as a second imaging device provided by the interventional imaging device.

According to an example, the first imaging device is provided as an X-ray imaging device. The second imaging device is provided as at least one of the group of: bronchoscope, endoscope, colonoscope, intravascular ultrasound, intracardiac echocardiography, endobronchial ultrasound or radial endobronchial ultrasound, and optical coherence tomography.

According to the present invention, also a method for guiding an interventional imaging device is provided. The method comprises the following steps:
- Providing first image data as first data from a first imaging device. The first image data comprises a representation of the interventional imaging device inserted within a vessel structure of a subject. The first image data comprises image data relating to a first point in time.
- Estimating a pose of the interventional imaging device in the first image data.
- Providing second data relating to a movement of the interventional imaging device. The second data relates to the first point in time and to at least a second point in time.
- Tracking a relative motion of the interventional imaging device based on the second data.
- Computing an updated pose estimate of the interventional imaging device based on the estimated pose and the tracked relative motion.
- Generating an updated indicator of the interventional imaging device based on the computed updated pose estimate.
- Augmenting the first image data with the updated indicator.
- Providing the augmented first image data.

According to an aspect, camera tracking or camera pose estimates is combined with the most recent fluoroscopy image acquired during the procedure, to augment the fluoroscopy image with an updated bronchoscope pose as the bronchoscope is navigated through the patient anatomy. This augmented view can help orient physicians and reduce their reliance on fluoroscopy images to orient themselves, therefore, reducing exposure to radiation. While the summary and description are provided relating to pulmonary lesions and bronchoscopic navigation to these lesions, the solution is also applicable to other endoscopic procedures that use fluoroscopy as well as procedures that use other devices (EBUS, IVUS) in combination with fluoroscopy.

According to an aspect, a setting is described that provides an imager with a larger field of view, e.g. the X-ray based imaging, plus an interventional device with an imager that has a small or local field of view within the lumen where the device is being navigated. The redundancy of using the two imaging techniques constantly, to visualize the same anatomy, is mostly resolved by tracking the camera on the device with a limited field of view and thus preventing users from becoming disoriented in complex anatomy.

As an effect, for instance, a bronchoscope's pose is updated on fluoroscopy images without continuous fluoroscopy image acquisition.

This results in the effect that an overreliance on fluoroscopy is avoided and the need for specialized tracked tools during interventional procedures is omitted. In addition to reducing radiation exposure, reducing reliance on fluoroscopy during interventional procedures can also result in reduced procedure time since, for instance, in pulmonary lesion biopsy procedures, users may not spend as much time repositioning the X-ray imaging system to acquire fluoroscopy images. Reducing reliance on expensive tracking devices and tracked tools also makes this solution more accessible and, therefore, makes safer procedures available for more patients.

This method can be used with any interventional imaging system including, but not limited to, fixed and mobile X-ray imaging systems during procedures with navigated imaging devices including, but not limited to, bronchoscopes, endoscopes and others.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for guiding an interventional imaging device.
Fig. 2 shows an example of a system for guiding an interventional imaging device.
Fig. 3 shows basic steps of an example of a method for guiding an interventional imaging device.
Fig. 4 shows an example of a workflow for guiding an interventional imaging device.
Fig. 5 shows an example of a further workflow for guiding an interventional imaging device.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

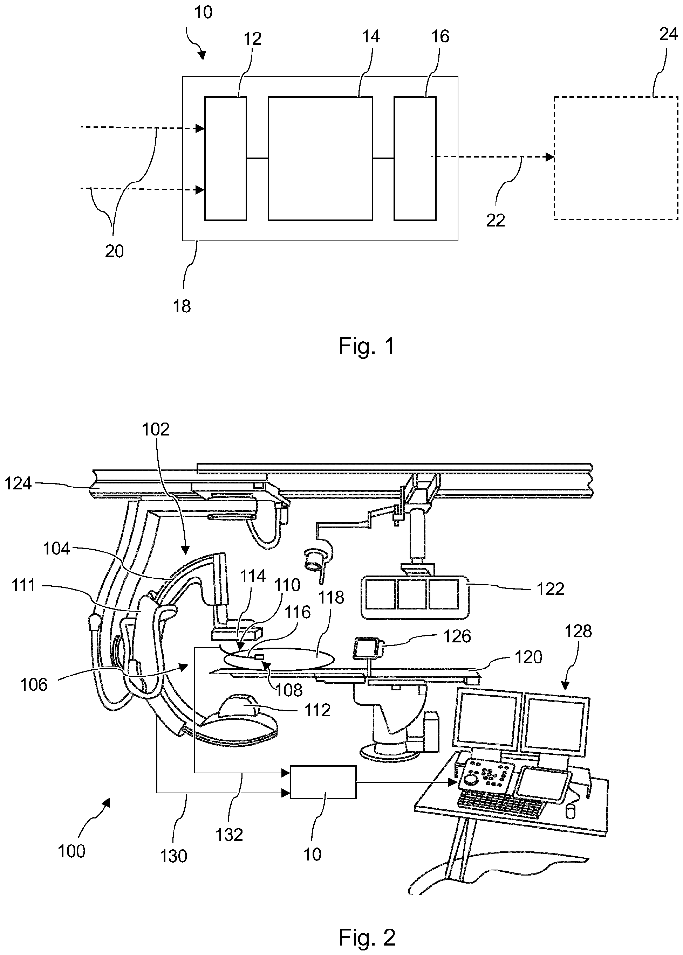

Fig. 1 schematically shows an example of a device 10 for guiding an interventional imaging device. The device 10 comprises a data input 12, a data processor and an output interface 16. The data input 12 is configured to provide first image data as first data from a first imaging device. The first image data comprises a representation of the interventional imaging device inserted within a vessel structure of a subject. Further, the first image data comprises image data relating to a first point in time. The data input 12 is also configured to provide second data relating to a movement of the interventional imaging device. The second data relates to the first point in time and to at least a second point in time. The data processor 14 is configured to estimate a pose of the interventional imaging device in the first image data. The data processor 14 is also configured to track a relative motion of the interventional imaging device based on the second data. The data processor 14 is further configured to compute an updated pose estimate of the interventional imaging device based on the estimated pose and the tracked relative motion. The data processor 14 is also configured to generate an updated indicator of the interventional imaging device based on the computed updated pose estimate. The data processor 14 is furthermore configured to augment the first image data with the updated indicator. The output interface 16 is configured to provide the augmented first image data.

The data input 12, the data processor 14 and the output interface 16 can be provided in a common structure, like a common housing, as indicated by a frame 18, or even in an integrated manner. In a further option (not shown), they are provided as separate components or units.

First arrows 20 indicate data supply to the data input 12, i.e. the provision of the first image data and the second data. A second arrow 22 indicates data supply from the output interface 16, i.e. the provision of the augmented first image data. The data-supplies can be provided wire-based or wireless.

In an example, as an option, a display 24 is provided to present the augmented first image. The display 24 is data-connected to the output interface 16.

The term "to estimate a pose" relates to assessing or determining the position and orientation of the interventional device, preferably the distal end of the interventional device, arranged within a vessel structure of the subject. As an example, the position and orientation are determined in relation to a projection of the first image data.

The term "relative motion" relates to a change in position and/or orientation of the interventional device arranged within the vessel structure relative to its position and/or orientation at the first point in time.

The term "updated pose estimate" relates to a further assessment or determination of the pose, i.e., the position and orientation of the interventional device, preferably its distal end.

The term "updated indicator" relates to an indicator reflecting the further assessment or determination. An indicator can be provided as a graphic element or illustration, or other visual means presented to the user.

The term "to augment" relates to providing additional information within the respective image. The image content is thus enhanced. The augmented image comprises more content than the image before the augmentation. While the initial image, i.e. the non-augmented image, comprises image data as seen by the respective image device, the augmented image provides additional content presented within the initial image.

The term "data input" relates to providing or supplying data for data processing steps. The data input 12 can also be referred to as image data input. The data input 12 can also be referred to as data supply, as image data supply, as image input, as input unit or simply as input. In an example, the image data input 12 is data-connectable to an imaging source arrangement. In an example, the data input 12 is data-connectable to a data storage having stored the image data.

The term "data processor" relates to a processor or part of a processor arrangement that is provided to conduct the computing steps using the data supplied by the data input. The data processor 14 can also be referred to as data processing arrangement, as processor unit or as processor. In an example, the data processor 14 is data-connected to the data input and the output interface.

The term "output interface" relates to an interface for providing the processed or computed data for further purposes. The output interface 16 can also be referred to as output or output unit. In an example, the output interface 16 is data-connectable to a display arrangement or display device. In another example, the output interface 16 is data-connected to a display.

In an example, for the updated indicator, a direction indicator is provided. For example, the direction indicator is provided as shading.

In an example, the first image data is provided as 2D image data. As an example, augmenting the first image data results in a 2D augmentation.

In an example, the second data is position and/or orientation data relating to the movement of the interventional imaging device. In other words, the second data is pose data. The data processor 14 is configured to track the relative motion of the interventional imaging device within the vessel structure based on the position and/or orientation data. In an option, the position and/or orientation data is provided as tracking data, like electromagnetic tracking. In another option, the position and/or orientation data is provided as shape sensing data of the interventional imaging device and as advancement information of the interventional imaging device.

In an option, the navigated imaging device is tracked using external hardware, e.g. electromagnetic "EM" tracking or shape sensing, so that image processing is not required to estimate the pose of the device (or its distal end). In this case, the controller needs to extract the device pose from the external hardware at the time that the fluoroscopy image is acquired in order to perform any correction of the two estimated poses. The remaining steps are performed as described above.
In another example, external tracking may be used in combination with image processing to estimate the pose of the interventional device.

In another example, the second data is image data from a second imaging device, i.e. second image data, provided by the interventional imaging device. The second image data comprises a representation of the interior within the vessel structure. The data processor 14 is configured to track the relative motion of the interventional imaging device within the vessel structure based on the second image data.

In case of imaging devices that capture RGB (red-blue-green color) images, this can be achieved using any of the various methods explored in the art that estimate the (absolute or relative) pose or motion of a device based on features or changes in features observed through sequences of images. For instance, in the case of bronchoscope tracking, traditional methods like structure from motion (SfM) methods or simultaneous localization and mapping (SLAM) methods as well as newer deep learning based methods for camera tracking may be used. Similar methods may also be used for tracking other navigated imaging devices. In the case of ultrasound based imaging devices, tracking may require branch detection or other methods to roughly localize the device within the patient anatomy.

As an example, the internal or navigated interventional imaging device can acquire images during an interventional procedure, such as: endoscope, bronchoscope, intravascular ultrasound (IVUS), intracardiac echocardiography (ICE), endobronchial ultrasound (EBUS) or radial endobronchial ultrasound (R-EBUS) and others.

As an option, a correction loop in-between the procedure is provided.

In a first example, the data processor 14 is further configured to provide the computing of the updated pose estimate comprising a correction of the out-of-plane pose estimate from the first image data using pose estimate from the second image data. In a second example, provided in addition or alternatively, the data processor 14 is further configured to provide the computing of the updated pose estimate comprising a correction of the in-plane pose estimate from the second image data using pose estimate from the first image data.

In an example, for the estimation of the pose of the interventional imaging device in the first image data, the data processor 14 is configured to use one or more of the images of the second image data provided as a plurality of images. The one or more images of the plurality of images are used to generate pose estimates that relate to at least the first point in time. The one or more images of the plurality of images is, therefore, used for adapting the estimated pose of the interventional imaging device in the first image data. Thus, the second data is used adapt or update the estimation performed using the first images. In other words, information from the second image domain is transferred to the first image domain. This transfer of information compensates lack of respective information in the first image data.

In an example, the image of the stream of second images shows an identifiable anatomical structure and a viewing direction can be estimated for the image, such that the viewing direction can be transferred to the first image.

In an example, the second image data comprises a stream of second images. The data processor 14 is configured to provide the tracking of the relative motion of the interventional imaging device for consecutive images of the stream of second images.

In an option, a visualization controller is provided configured to continually display the updated bronchoscope trajectory on the most recent fluoroscopy image.

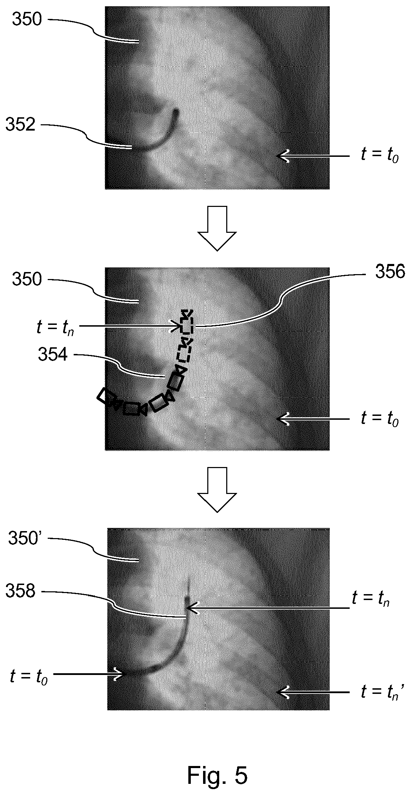

In an example, the data processor 14 is further configured to compute a trajectory of the interventional imaging device based on the updated pose estimate. In an option, the data processor 14 is further configured to generate, based on the computed trajectory and the updated pose of the interventional imaging device, a projection of the trajectory of the interventional imaging device; and to augment the first image data based on the projection of the trajectory of the interventional imaging device to provide an updated virtual first image data. In another option, the data processor 14 is further configured to project the computed trajectory onto the first image data (see also Fig. 5).

The computing of the trajectory is provided in addition to or instead of computing the updated pose estimate. The generating of the projection of the interventional imaging device is provided in addition to or instead of the generation of the updated indicator. The overlaying of the projection is provided as augmenting in addition to or instead of the augmenting the first image data with the updated indicator.

The updated virtual first image data can also be referred to as augmented first image data or artificial first image data.

In an option, the augmentation is provided inclusive of both overlaying lines or other shapes on top of the first image and modifying the first image using generative neural networks, for instance generative adversarial networks (see below).

In an example, the data processor is configured: to generate, based on the computed trajectory and the updated pose of the interventional imaging device, a projection of the interventional imaging device; and to overlay the projection of the interventional imaging device onto the first image data to provide an updated virtual first image data (see also Fig. 5).

According to an example (see also Fig. 5), data processor 14 is further configured to use a trained generative neural network to generate, based on the first image data at the first point in time and an updated pose of the interventional imaging device at a second point in time, a realistic synthetic image rendering the updated pose of interventional imaging device. The realistic synthetic image is then provided, e.g. presented to a user.

In an option, a generative neural network, for instance, a generative adversarial network (GAN), is used to generate fluoroscopy images with updated bronchoscope pose as the bronchoscope is navigated. This can be achieved by performing the same steps described above with the addition of a trained GAN inserted after the projection takes the augmented fluoroscopy image with an overlay of the updated bronchoscope pose and produces a new fluoroscopy image with a realistic bronchoscope rendering at its updated pose (as shown in Fig. 5, lower part). When the next fluoroscopy image is acquired, this rendering can be updated as described above. This avoids or at least reduces user distraction that overlays may cause, i.e., to avoid user distraction from anatomical features of importance in the fluoroscopy image.

In an example, the first image data provides a first field of view, and the second image data provides a second field of view. The first field of view is larger than the second field of view. As an example, the first field of view is at least twice as large as the second field of view.

In an example, the first image data comprises X-ray image data. The second image data comprises at least one of the group of: optical camera image data, ultrasound image data and optical coherence tomography image data.

In an example, the data processor 14 is further configured to provide a confidence estimate related to the relative motion estimate from the second image data. The output interface 16 is configured to provide a confidence indicator to the user.

In an option, a confidence estimate is provided along with the pose estimate from bronchoscopy. Confidence estimates can be made using any method available in the art and can be based on, for instance, the quality of images used to estimate pose, e.g. low confidence if image contains blur, specular reflections, etc., and/or the confidence of the pose estimation method itself. For instance, in a neural network based method, confidence in pose estimation may be evaluated using dropout layers in the network which randomly drop the outputs of a specified number of nodes in the network. When relative pose estimates between a particular pair of images are then computed multiple times, the network produces slightly different results due to the influence of slightly different nodes on the output. The mean and standard deviation of the results can be computed. If the network is confident in its pose estimate, the standard deviation will be small, while standard deviation will be large when the network is less confident. If confidence in relative pose estimate between two frames is low, then the pose estimate may be replaced by an extrapolation from the previous few poses. This low confidence or replacement of pose estimate may be visualized with a different color or transparency and can be an indicator to users on when a new fluoroscopy image may need to be acquired.

In an example, the data processor 14 is further configured to base the tracking of the relative motion of the interventional imaging device within the vessel structure on further tracking data. As an option, the tracking of the relative motion is done by referring to other data than imaging data plus the imaging data as the second data. As another option, the tracking of the relative motion is done by referring only to other data than imaging data, i.e. without the imaging data as the second data.

In an example, for displaying the device at a starting point in time *to,* X-ray images are used. At a further point in time *t₁,* or other further points in time *tₙ,* bronchoscopy information is provided and the derived position is added to the X-ray image.

As an example, the fluoroscopy image processing controller takes the most recent fluoroscopy image or sequence of images visualizing the navigated imaging device within patient anatomy and estimates the pose of the navigated imaging device tip (or distal end). This can be achieved using any of the various methods explored in the art that estimate the 2D or 3D pose of the imaged device such that its projection matches that seen in the fluoroscopy image(s).

In some cases, the 3D model of the device is known and is deformed and fitted to a configuration that matches its projection in fluoroscopy. In other cases, the 3D model may not be known, and the pose of a skeletonized distal end is estimated based on image features.

The image processing controller may use machine learning or deep learning techniques. Since fluoroscopy images are 2D projection images, the estimated pose may contain inaccuracies.

As an example, the image processing controller, or the data processor 14, takes in the stream of frames produced by the navigated imaging device, like the bronchoscope, as it is being navigated through patient anatomy and estimates the relative pose or motion produced by the navigated device.

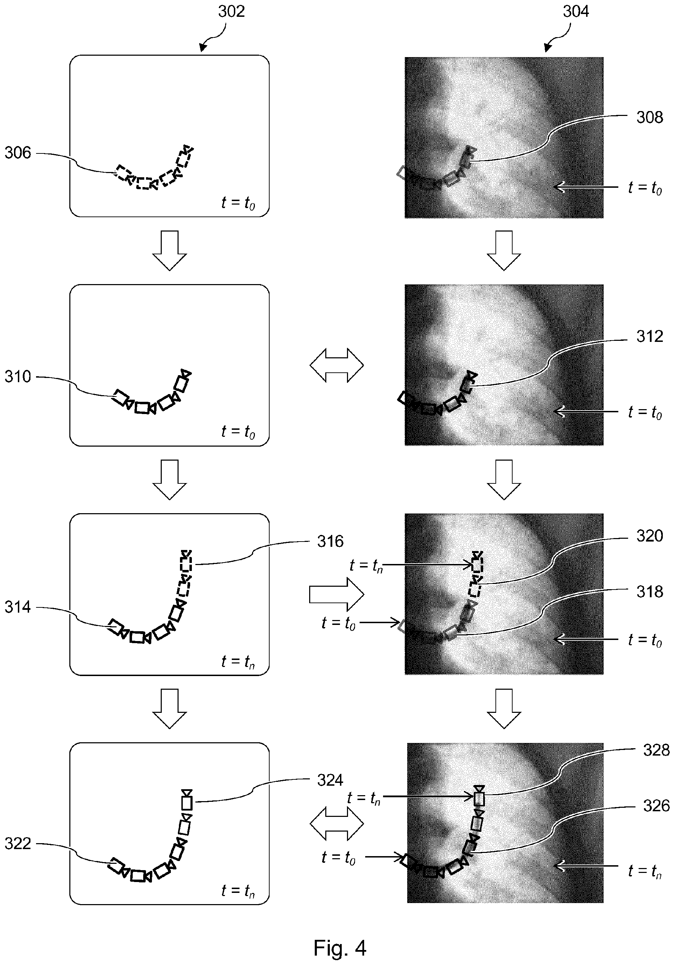

In an example, a controller is provided as the data processor 14. The controller is configured to perform the following tasks (the following refers to bronchoscopy as an example only):
- Estimation of the pose of the bronchoscope in a most recent fluoroscopy image acquired at *t = t₀.* As an example, a fluoroscopy image processing controller is provided as described below;
- Estimation of the relative motion of the bronchoscope between consecutive frames. As an example, an image processing controller is provided as described below.
- Usage of the two estimations together, to provide a correction of an out-of-plane pose estimate from fluoroscopy using pose estimated from bronchoscopy and correction of the in-plane pose estimate from bronchoscopy using pose estimate from fluoroscopy, shown using the bidirectional arrow in the second panel from top in Fig. 4).
- Continuation of the estimation of relative motion of bronchoscope between consecutive frames as the bronchoscope moves past its position at *t* = *t₀* and to sequentially add these estimates to the pose of the device at *t = t₀* to compute the trajectory of the bronchoscope (shown in the third panel from top in Fig. 4).
- Projection of the computed trajectory onto the fluoroscopy image plane to augment the most recent fluoroscopy image with the updated bronchoscope pose.
- If or when the next fluoroscopy image is acquired at *t* = *to,* this process of combining estimated bronchoscope pose from fluoroscopy and bronchoscopy and updating the new fluoroscopy image with bronchoscope trajectory as the bronchoscope continues to move can be repeated (shown in the lower panel in Fig. 4).

Fig. 2 shows an example of a system 100 for guiding an interventional imaging device. The system 100 comprises a first data arrangement 102 comprising a first imaging device 104 and a second data arrangement 106. Further, the system 100 comprises an example of the device 10 for guiding an interventional imaging device according to any of the preceding and following examples. The first imaging device 104 is configured to generate the first image data as the first data. The second data arrangement 106 is configured to generate the second data.

In one option, the second data arrangement 106 comprises a second imaging device 108. In another option (not shown), the second data arrangement 106 comprises a tracking data device or positioning data device.

In an example, an interventional imaging device 110 is provided. As an option, the second data arrangement 106 provided as the second imaging device 108 is provided by, i.e. mounted on, the interventional imaging device.

In an example, as shown in Fig. 2, the first imaging device 104 is provided as an X-ray imaging device 111 with an X-ray source 112 and an X-ray detector 114 mounted to a movably supported C-arm. The second imaging device is provided as bronchoscope 116. In further examples, the second imaging device is provided as at least one of the group of: endoscope, colonoscope, intravascular ultrasound, intracardiac echocardiography, endobronchial ultrasound or radial endobronchial ultrasound, optical coherence tomography.

A subject 118, for example a patient, is arranged on a subject support 120. An arrangement 122 is supported by a ceiling-mounted rail structure 124. Further, a control interface 126 is arranged next to the subject support 120.

A console 128 is provided in the foreground. The console 128 comprises displays, keyboard, mouse, graphic tablet, control knobs and the like for providing user interaction and control options. The console is provided for controlling the various functions and operations of the system 100 for guiding an interventional imaging device. The device 10 for guiding an interventional imaging device can be arranged integrated in the console or as separate device. The device 10 for guiding an interventional imaging device is data-connected to the first data arrangement 102, as indicated with a first data connection line 130. The device 10 for guiding an interventional imaging device is also data-connected to the second data arrangement 106, as indicated with a second data connection line 132. The data-connection is provided wire-based or wireless.

The bronchoscope 116 is inserted into the subject 118 and the X-ray imaging device 111 can provide fluoroscopy images of the subject 118 as the first image data, i.e. the first images. A representation of the bronchoscope 116 is visible in the X-ray image due to differing X-ray attenuation characteristics of the bronchoscope 116 in relation to the surrounding anatomical structures. The bronchoscope 116 provides bronchoscopy images as the second image data, i.e. as the second images.

The X-ray images are used to estimate a pose of the interventional imaging device at a first point in time. The bronchoscopy images are used to track a relative motion of the interventional imaging device. An updated pose estimate of the interventional imaging device is computed based on the estimated pose (in the X-ray images) and the tracked relative motion (in the bronchoscopy images). An updated indicator of the interventional imaging device is generated based on the computed updated pose estimate. The X-ray image is augmented with the updated indicator. The augmented X-ray image is presented to the user, for example on the display arrangement 122.

In one option, the updated indicator comprises a graphic representation of the interventional imaging device with an indication of the bronchoscope's camera viewing direction. The indication may be provided in the form of an overlay. This provides the advantage of an improved user understanding of the current situation. The indication of the bronchoscope's camera viewing direction provides information on the spatial arrangement, i.e. 3D information, of the current situation in an intuitive way. However, a current X-ray projection is not required, meaning a reduction / minimization in radiation dose.

In another option, the updated indicator comprises a simulated X-ray projection of the interventional imaging device. In this option, a generative neural network or other technique may be used to generate, based on the initial X-ray image with the initial pose of the interventional imaging device and on the updated pose of the interventional imaging device, an updated X-ray image without X-ray image acquisition. This provides an understanding of the current situation without the dose of an extra or additional X-ray image. The simulated projection mimics the additional image and provides a non-distracting and intuitive source of information for the user.

In an example, a system is provided that comprises an external interventional imaging system, an internal or navigated interventional imaging device, a fluoroscopy image processing controller and an image processing controller.

In an example, pose estimation from a navigated imaging device, e.g. a bronchoscope, an endoscope, EBUS, IVUS, etc., and the most recent image from a secondary imaging device with a larger FOV that can image the navigated device within the patient anatomy, e.g. X-ray, CBCT, tomosynthesis, etc., is combined in order to update the pose of the navigated imaging device in the most recent image from the secondary imaging device.

Pose estimation in the context of the present disclosure refers to estimating the position and viewing angle of the imaging device.

According to an aspect, it is provided to estimate a first position from a first imaging system at regular intervals, and to estimate a second position from an intervention device during the intervals and to update the first position with the second position.

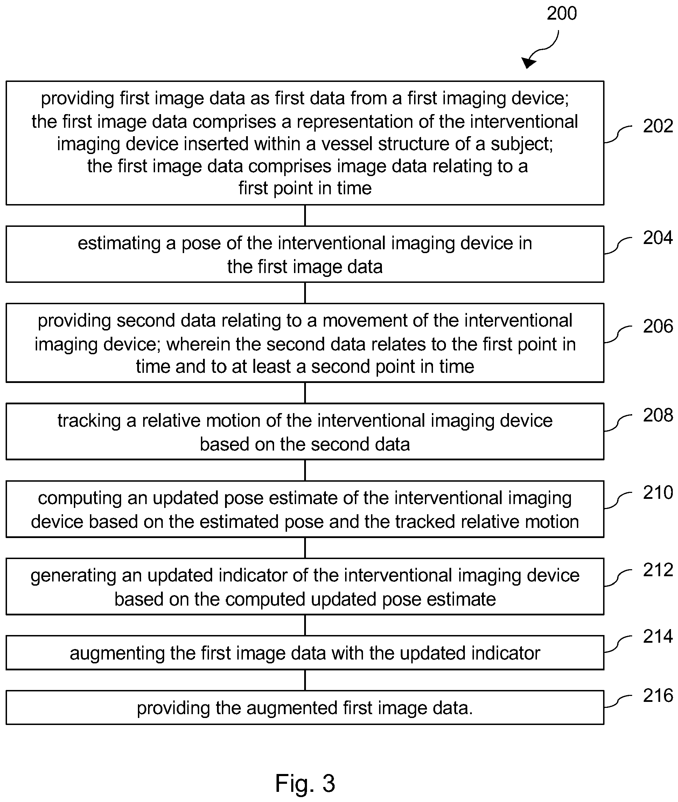

Fig. 3 shows basic steps of an example of a method 200 for guiding an interventional imaging device. The method 200 comprises the following steps:
- In a first step 202, first image data is provided as first data from a first imaging device. The first image data comprises a representation of the interventional imaging device inserted within a vessel structure of a subject. The first image data comprises image data relating to a first point in time.
- In a second step 204, a pose of the interventional imaging device in the first image data is estimated.
- In a third step 206, second data relating to a movement of the interventional imaging device is provided. The second data relates to the first point in time and to at least a second point in time.
- In a fourth step 208, a relative motion of the interventional imaging device is tracked based on the second data.
- In a fifth step 210, an updated pose estimate of the interventional imaging device is computed based on the estimated pose and the tracked relative motion.
- In a sixth step 212, an updated indicator of the interventional imaging device is generated based on the computed updated pose estimate.
- In a seventh step 214, the first image data is augmented with the updated indicator.
- In an eighth step 216, the augmented first image data is provided.

In an example of the method, the first image data is provided as 2D image data.

In an example of the method 200, the interventional imaging device comprises a second imaging device. The second data is provided as second image data from the second imaging device. The second image data comprises a representation of the interior within the vessel structure. The tracking of the relative motion of the interventional imaging device within the vessel structure is based on the second image data.

In an example of the method 200, for the estimation of the pose of the interventional imaging device in the first image data, one or more images of the stream of second images used to generate pose estimates relating to at least the first point in time are used for adapting the estimated pose of the interventional imaging device in the first image data.

In an example of the method 200, the second image data comprises a stream of second images. The tracking of the relative motion of the interventional imaging device is provided for consecutive images of the stream of second images.

In an example of the method 200, it is further provided the steps of: computing a trajectory of the interventional imaging device based on the updated pose estimate; generating, based on the computed trajectory and the updated pose of the interventional imaging device, a projection of the trajectory of the interventional imaging device; and augmenting the first image data based on the projection of the trajectory of the interventional imaging device onto the first image data to provide an updated virtual first image data.

In another example of the method 200, it is further provided the steps of: computing a trajectory of the interventional imaging device based on the updated pose estimate; and projecting the computed trajectory onto the first image data.

In an example of the method 200, the computing of the updated pose estimate comprises at least one of the group of: a correction of the out-of-plane pose estimate from the first image data using pose estimate from the second image data; and a correction of the in-plane pose estimate from the second image data using pose estimate from the first image data.

In another example of the method 200, the first image data comprises X-ray image data. The second image data comprises at least one of the group of: optical camera image data, ultrasound image data and optical coherence tomography image data.

In an example of the method 200, a confidence estimate related to the relative motion estimate from the second image data is provided. A confidence indicator is provided to the user.

In another example of the method 200, the tracking of the relative motion of the interventional imaging device within the vessel structure is based on further tracking data.

Fig. 4 shows a workflow of an example for guiding an interventional imaging device. In a left column 302, information from a bronchoscope as an example of an interventional device as the second data arrangement 106 is provided. The left column relates to the second data provided by the second imaging device 108, for example in form of the bronchoscope 116. In a right column 304, information from an X-ray imager as the first data arrangement 102 is provided. The right column relates to the first data provided by the first imaging device 104, for example in form of the X-ray imaging device 111. As indicated, an estimated relative camera motion 306 at *t* = *t₀* is derived from bronchoscope data. Further, an extracted bronchoscope pose 308 is derived from fluoroscopy image at *t* = *to.* Next, a corrected relative camera motion 310 at *t* = *t₀* is determined. A corrected bronchoscope pose 312 at *t* = *t₀* is generated. Further, from bronchoscope, a relative camera motion 314 at *t = tₙ* is determined, *n>0.* An added part 316 of the bronchoscope indicator indicates the movement of the bronchoscope. This provides for an updated bronchoscope overlay 318 in fluoro, also indicating the part of the bronchoscope from the initial state plus the added part 320 from the movement of the bronchoscope. Next, if an updated fluoroscopy image is acquired at *t* = *tₙ,* a corrected relative camera motion at *t = tₙ* is provided. An added part 324 of the bronchoscope indicator represents the corrected relative camera motion, while a first part 322 indicates the bronchoscope from the initial state. An extracted / updated bronchoscope pose from fluoroscopy image at *t = tₙ* is also provided, with an initial part 326 and an added part 328.

The second image data from the bronchoscope 116 is provided from at least two points in time: a first, e.g. initial or starting point in time, *t = t₀*; and a second, e.g. later point in time, *t* = *tₙ.*

The first image data from the X-ray imaging device 111 is provided from the first, e.g. initial or starting point in time, *t* = *t₀.*

The augmented first image data as shown in the third column is generated and provided at the second, e.g. later point in time, *t* = *tₙ,* but literally only with respect to the augmented part in which the indicator is overlaid. Since the augmented first image data as shown in the third column is based on the first image data from the X-ray imaging device 116 taken at the first, e.g. initial or starting point in time, *t* = *t₀,* the other parts relate to that first, e.g. initial or starting point in time, *t* = *t₀.*

In Fig. 4, the single arrows between the left and right columns indicate a forwarding or transferring of data and information. Within the same column, the single arrows indicate a processing of data. The double arrows indicate a mutual use or mutual transferring of data and information between the two domains, i.e. the two coordinate frames of the bronchoscope images and the X-ray images.

Fig. 5 shows a workflow of another example for guiding an interventional imaging device. A most recent fluoroscopy image 350 is provided in the upper field, in which a bronchoscope 352 is shown. An updated bronchoscope pose 356 of a bronchoscope 354 is provided in the middle field. Here, the updated bronchoscope pose 536 is overlaid onto the fluoroscopy image in the middle field. Thus, further information derived from another domain, e.g. the bronchoscope domain, is added to the domain of the fluoroscopy image. The updated bronchoscope pose 356 is used to generate a simulation of a projection of the interventional imaging device in the respective pose. The simulation may directly generate a realistic fluoroscopy image with an updated pose of the bronchoscope as shown in the lower field. This may be done using generative neural networks that are trained to generate realistic renderings of devices within anatomy given an updated device pose. As a result, a generated fluoroscopy image 350' with an updated bronchoscope pose 358 is generated. The generated new image is supposed to represent an image at t = tₙ*.* In Fig. 5, this is indicated with t = tₙ', where the ' represents the fact that it is a simulated or synthetic image.

The first image data from the X-ray imaging device 116 is provided from the first, e.g. initial or starting point in time, *t* = *t₀.* Hence, the fluoro image in the upper part of Fig. 5 is from the first, e.g. initial or starting point in time, *t* = *t₀.*

The second image data from the bronchoscope 116 is provided from at least a a second, e.g. later point in time, *t* = *tₙ.*

The added fluoro image in the middle part of Fig. 5 is generated and provided at the second, e.g. later point in time, *t* = *tₙ,* but literally only with respect to the overlaid pose of the bronchoscope. Since the augmented first image data as shown in the middle field is based on the first image data from the X-ray imaging device 116 taken at the first, e.g. initial or starting point in time, *t* = *t₀,* the other parts relate to that first, e.g. initial or starting point in time, *t* = *t₀.*

The augmented first image data as shown in the lower field is generated and provided at the second, e.g. later point in time, *t* = *tₙ,* but literally only with respect to the augmented part in which the simulation generates the updated bronchoscope rendering. Since the augmented first image data as shown in the lower field is based on the first image data from the X-ray imaging device 116 taken at the first, e.g. initial or starting point in time, *t* = *t₀,* the other parts relate to that first, e.g. initial or starting point in time, *t* = *t₀.* However, since the image is generated based on an estimate of the bronchoscope pose at a later point in time, *t* = *tₙ,* without X-ray image acquisition, the image is denoted as having been generated at time *t* = *tₙ'.*

In Fig. 5, the single arrows indicate a forwarding or transferring of data and information with a processing of data.

The term "subject" may also be referred to as individual. The "subject" may further also be referred to as patient, although it is noted that this term does not indicate whether any illness or disease is actually present with the subject.

In another example, a computer program comprising instructions which, when the program is executed by a computer, cause the computer to carry out the method of one of the preceding examples.

In an example, a computer program or program element for controlling an apparatus according to one of the examples above is provided, which program or program element, when being executed by a processing unit, is adapted to perform the method steps of one of the method examples above.

In another example, a computer readable medium having stored the computer program of the example above is provided.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for guiding an interventional imaging device, comprising:
- a data input (12);
- a data processor (14); and
- an output interface (16);
wherein the data input is configured: to provide first image data as first data from a first imaging device, wherein the first image data comprises a representation of the interventional imaging device inserted within a vessel structure of a subject, and wherein the first image data comprises image data relating to a first point in time; and to provide second data relating to a movement of the interventional imaging device, wherein the second data relates to the first point in time and to at least a second point in time;
wherein the data processor is configured: to estimate a pose of the interventional imaging device in the first image data; to track a relative motion of the interventional imaging device based on the second data; to compute an updated pose estimate of the interventional imaging device based on the estimated pose and the tracked relative motion; to generate an updated indicator of the interventional imaging device based on the computed updated pose estimate; and to augment the first image data with the updated indicator; and
wherein the output interface is configured to provide the augmented first image data.

2. Device according to claim 1, wherein the second data is second image data from a second imaging device provided by the interventional imaging device;
wherein the second image data comprises a representation of the interior within the vessel structure; and
wherein the data processor is configured to track the relative motion of the interventional imaging device within the vessel structure is based on the second image data.

3. Device according to one of the preceding claims, wherein the data processor is further configured: to provide the computing of the updated pose estimate comprising at least one of the group of:
- a correction of the out-of-plane pose estimate from the first image data using pose estimate from the second image data; and
- a correction of the in-plane pose estimate from the second image data using pose estimate from the first image data.

4. Device according to claim 2 or 3, wherein, for the estimation of the pose of the interventional imaging device in the first image data, the data processor is configured to use one or more images of the second image data used to generate pose estimates relating to at least the first point in time for adapting the estimated pose of the interventional imaging device in the first image data.

5. Device according to claim 2, 3 or 4, wherein the second image data comprises a stream of second images; and
wherein the data processor is configured to provide the tracking of the relative motion of the interventional imaging device for consecutive images of the stream of second images.

6. Device according to one of the preceding claims, wherein the data processor is further configured: to compute a trajectory of the interventional imaging device based on the updated pose estimate; and
i) to generate, based on the computed trajectory and the updated pose of the interventional imaging device, a projection of the trajectory of the interventional imaging device; and to augment the first image data based on the projection of the trajectory of the interventional imaging device to provide an updated virtual first image data; or
ii) to project the computed trajectory onto the first image data.

7. Device according to one of the preceding claims, wherein the data processor is further configured to use a trained generative neural network to generate, based on the first image data at the first point in time and an updated pose of the interventional imaging device at a second point in time, a realistic synthetic image rendering the updated pose of interventional imaging device.

8. Device according to one of the preceding claims, wherein the first image data comprises X-ray image data; and
wherein the second image data comprises at least one of the group of: optical camera image data, ultrasound image data and optical coherence tomography image data.

9. Device according to one of the preceding claims, wherein the data processor is further configured to provide a confidence estimate related to the relative motion estimate from the second image data; and
wherein the output interface is configured to provide a confidence indicator to the user.

10. A system (100) for guiding an interventional imaging device, the system comprising:
- a first data arrangement (102) comprising a first imaging device (104);
- a second data arrangement (106); and
- a device (10) for guiding an interventional imaging device according to any of the preceding claims;
wherein the first imaging device is configured to generate the first image data as the first data; and wherein the second data arrangement is configured to generate the second data.

11. System according to claim 10, wherein an interventional imaging device (110) is provided; and
wherein the second data arrangement is provided as a second imaging device provided by the interventional imaging device.

12. System according to claim 10 or 11, wherein the first imaging device is provided as an X-ray imaging device (111); and
wherein the second imaging device is provided as at least one of the group of: bronchoscope (116), endoscope, colonoscope, intravascular ultrasound, intracardiac echocardiography, endobronchial ultrasound or radial endobronchial ultrasound, and optical coherence tomography.

13. A method (200) for guiding an interventional imaging device, comprising the following steps:
- providing (202) first image data as first data from a first imaging device; wherein the first image data comprises a representation of the interventional imaging device inserted within a vessel structure of a subject; wherein the first image data comprises image data relating to a first point in time;
- estimating (204) a pose of the interventional imaging device in the first image data;
- providing (206) second data relating to a movement of the interventional imaging device; wherein the second data relates to the first point in time and to at least a second point in time;
- tracking (208) a relative motion of the interventional imaging device based on the second data;
- computing (210) an updated pose estimate of the interventional imaging device based on the estimated pose and the tracked relative motion;
- generating (212) an updated indicator of the interventional imaging device based on the computed updated pose estimate;
- augmenting (214) the first image data with the updated indicator; and
- providing (216) the augmented first image data.

14. A computer program comprising instructions which, when the program is executed by a computer, cause the computer to carry out the method of claim 13.

15. A computer readable medium having stored the computer program of claim 14.
